Europäisches Patentamt

European Patent Office

Office européen des brevets

(11) Publication number: **0 226 427 B1**

# EUROPEAN PATENT SPECIFICATION

(45) Date of publication of patent specification: **17.07.91** (51) Int. Cl.⁵: **C12Q 1/32**, G01N 33/04

(21) Application number: **86309548.5**

(22) Date of filing: **08.12.86**

(54) **Method for simultaneously inspecting the quality of the milk secreted by an animal and diagnosing its ketosis state.**

(30) Priority: **10.12.85 JP 276048/85**

(43) Date of publication of application:
**24.06.87 Bulletin 87/26**

(45) Publication of the grant of the patent:
**17.07.91 Bulletin 91/29**

(84) Designated Contracting States:
**CH DE FR GB IT LI**

(56) References cited:
**EP-A- 0 140 589**
**EP-A- 0 149 853**
**DE-A- 3 408 062**

**CHEMICAL ABSTRCATS, vol. 104, no. 17, 28th April 1986, page 554, column 2, abstract no. 147255m, Columbus, Ohio, US; S. RAJAMAKI et al.: "The determination of acetoacetate and 3-hydroxybutyrate in milk"**

(73) Proprietor: **SANWA KAGAKU KENKYUSHO CO., LTD.**
**No. 35, Higashi-sotobori-cho**
**Higashi-ku Nagoya-shi Aichi-ken(JP)**

(72) Inventor: **Asai, Hiromoto**
**5-1-6, Nakayama-cho Mizuho-ku**
**Nagoya-shi Aichi-ken(JP)**
Inventor: **Asai, Masaru**
**925, Aza-waseda Ohaza-kumahari Nagakute-cho**
**Aichi-gun Aichi-ken(JP)**
Inventor: **Ito, Toshio**
**17, Aza-shiroshita Ohaza-obata Moriyama-ku**
**Nagoya-shiŠAichi-ken(JP)**
Inventor: **Fujii, Tokio**
**Sanny Building 303 5-4, Yasudatori Showa-ku**
**Nagoya-shi Aichi-ken(JP)**
Inventor: **Furukawa, Eiji**
**2-9-41, Shindeki Higashi-ku**
**Nagoya-shi Aichi-ken(JP)**

(74) Representative: **Bubb, Antony John Allen et al**
**GEE & CO. Chancery House Chancery Lane**
**London WC2A 1QU(GB)**

## Description

The present invention relates to a method for inspecting the quality of milk secreted by an animal and simultaneously diagnosing whether the animal is in a state of ketosis.

The health inspection of an animal secreting milk such as dairy cattle is quite important to protect a mother body of such animal and a young animal from a various kind of diseases and to obtain milk of a high quality by milking. Under such circumstances, different kinds of techniques for such health inspection of animals secreting milk have been proposed and put into practical use.

One of such inspection techniques performed during the health inspection of, for example, dairy cattle comprises quantitatively analyzing the amount of ketones contained in blood and urine thereof. More particularly, the ketones include acetone, acetoacetic acid, $\beta$-hydroxybutyric acid or the like which are excessively accumulated in tissues and blood or excreted in urine. The ketones are formed due to the oxidation of fatty acids in the liver which is activated when an incompleteness in metabolism of carbohydrates is caused due to decrease in the amount of blood sugar accompanied by the increase in the amount of milk secretion. Under such condition, the animal is in the so-called ketosis state which is liable to be caused in mother animals at the end of gestation period or after the parturition and which causes inappetence and the decrease in milk secretion, in the worst case paralysis being caused. Therefore, the quantitative analysis of ketones in blood an urine is quite important and makes it possible to provide an indication for judging whether the inspected animal suffers from ketosis or not.

The conventional quantitative analysis of ketones as described by Todd et al in "Clinical Diagnosis and Management by Laboratory Methods", Vol 1, page 594 (1979), and as carried out on dairy cattle comprises examining the amount of acetoacetic acid in a sample to be inspected such as blood or urine, in particular urine. In the method for quantitatively analyzing the amount of acetoacetic acid in a blood or urine as the specimen for diagnozing whether dairy cattle suffers from ketosis or not according to a conventional technique mentioned above, such quantitative analysis is usually carried out utilizing an indicating means such as indicator paper used for measuring human ketones. The indicating means is obtained by treating the test paper portion thereof with sodium nitroprussideglycine-alkali buffer and its sensitivity to acetoacetic acid is in a range of from 5 to 10 mg/dl.

However, the acetoacetic acid to be quantitatively analyzed according to the conventional method is a material having a quite low stability and therefore, the acetoacetic acid included in a specimen is easily and spontaneously converted to acetone, thereby evaporating and escaping in the air if the specimen is left to stand even for a short time. This means that the specimen for detecting ketosis should always be a fresh one and, for example, if the sample is urine, it is required to withdraw urine from the urocystis utilizing an urethral catheter. However, the withdrawal of urine relies heavily on a skilled person such as a veterinary and moreover there is a risk for causing infectious diseases such as urethritis, urocystitis or the like if the withdrawal of urine is not effected with care. When the veterinary carries out the withdrawal of urine with respect to the dairy cattle which belongs to the dairy farmer affiliating with the agricultural mutual benefit, it is stipulated to pay a consideration of the withdrawal of urine to the veterinary. However, the amount thereof does not sufficiently compensate for such special technical works. As a result, the withdrawal of urine for obtaining fresh urine as the specimen for diagnosis of ketosis is seldom carried out and actually, a specimen collected by the owner of the dairy cattle during the usual urination is provided for the inspection. This results in the reduction of reliability of the test results as well as the increase in the owner's burden because of the low freshness of the specimen.

Moreover, when an animal milk, for instance, milk of dairy cattle is forwarded to an inspecting office, it is subjected to quality inspections such as alcohol test and acidity test to classify the milk according to quality thereof. In such case, if milk from an animal suffering from ketosis is milked and forwarded to the inspecting office without knowing it, the milk is possibly judged to be low quality as a result of the quality inspection explained above. At this stage, it is quite difficult or rather impossible to identify the animal suffering from ketosis and it is a matter of course that the milk from such animal cannot be separated from those normal animal. In this case, therefore, it is necessary to carry out health inspection on whole animals subjected to milking at that time for the purpose of the identification of the animal in a bad physical condition and to give them a suitable medical treatment, if the past records of the whole animals milked are good.

Furthermore, other than the reduction in the quality of milk which leads to the reduction of the price thereof, the capacity of secreting milk of an animal is also remarkably influenced by ketosis, in particular in the case of dairy cattle. This is one of the most important problem to dairy farmers. In other words, ketosis tends to frequently occur during the term of from one week to three months after parturition, thereby the capacity of milk secretion being significantly reduced. For instance, it has been reported that 25% reduction in the amount of milk secretion is observed in the worst case, although the reduction thereof may vary depending on the condition of ketosis. Moreover, it is often observed to cause reproductive difficulties when the term during which ketosis frequently occurs and that for the subsequent mating are overlapped with each other and the animal suffers from the ketosis. As seen from the above, the influence of ketosis on the management of dairy farming is quite enormous.

Thus, it is quite desirable to previously confirm quality of milk in some extent before carrying out the practical milking operations in order to prevent such troublesome situation explained above from occurring preliminarily and for that purpose, the development of a simple method for inspecting quality of an animal milk as a specimen and health of the animal secreting the milk, in particular dairy cattle, has been desired for a long time.

Our prior EP-0140589 describes a method of enzymatic determination of D-3-hydroxy butyric acid in a sample of mammalian body fluid such as blood, comprising converting the acid into acetoacetic acid in the presence of nicotinamide adenine dinucleotide and a 3-hydroxybutyric denhydrogenase and a lactic dehydrogenase inhibitor, and measuring the resultant color. There is no suggestion of using this method on a sample of milk, nor for diagnosis of ketosis.

DE-3408062 discloses a process for determining a betahydroxybutyric acid, in which the acid is oxidised in the presence of nicotinamide adenine dinucleotide by use of an electron carrier and a tetrazolium salt, and the resultant color is again measured. The invention is useful in diagnosising diabetes.

Therefore a principal object of the present invention is to provide a simple method for simultaneously inspecting the quality of the milk secreted by an animal and diagnosing whether the animal is in a state of ketosis, and preferably to provide such method that can be carried out rapidly, simply and without a skilled technician.

The invention provides a new method in which the specimen (the sample to be examined) is the milk of the animal, because the collection of milk is relatively easy and its freshness is relatively high, and beta-hydroxybutyric acid is selected as the ketone to be quantitatively analysed because of its high stability.

According to the present invention we provide a method for simutaneously inspecting the quality of the milk secreted by an animal and diagnosing whether the animal is in a state of ketosis, which comprises collecting milk (a small amount thereof is adequate) from the animal to be examined, dehydrogenating $\beta$-hydroxybutyric acid in the sample with a $\beta$-hydroxybutyric acid dehydrogenase in the presence of nicotinamide adenine dinucleotide (NAD) to convert the same to acetobutyric acid, adding and reducing a tetrazolium salt with the nicotinamide adenine dinucleotide in the reduced form (NADH), formed during the dehydrogenation step, in the presence of an electron carrier to convert the tetrazolium salt to colored formazan and then determining the amount of $\beta$-hydroxybutyric acid included in the specimen based on the degree of color development or color tone of the reaction system.

In a preferred embodiment of the present invention, the determination of the amount of $\beta$-hydroxybutyric acid in the speciment is performed by macroscopically comparing or contrasting the color tone developed by the resulting formazan with those in a preliminarily determined standard color tone table.

Another preferred embodiment according to the present invention comprises directly treating or mixing a sample, for instance, milk of dairy cattle with a mixture containing a $\beta$-hydroxybutyric acid dehydrogenase, NAD, an electron carrier and a tetrazolium salt, which may be in a liquid form or in a solid form.

When the mixture is used in the liquid form, a solvent, for instance, a buffer solution such as phosphate buffer, borate buffer, Tris buffer, Good's buffer may be used and preferably the solvent used has a pH value corresponding to those optimum for the enzyme reaction and color reaction (for example, pH 7 to 10) taking place during the inspection procedures. Moreover, the mixture may include other additives, for example, a stabilizer such as serum albumin.

On the other hand, if the mixture is used in the solid form, it may be applied to the surface of a support material, for example, absorbing supports such as a filter paper and plastic sheets or films such as polyester film. In the mixture of the reagents, a binder such as gelatin, may also be added to improve the adhesiveness of the mixture to the support.

For carrying out the method of the invention there can preferably be used an indicator which comprises a support having absorbed therein or in a layer applied thereon, the above mentioned four components.

The indicator for use in the present method may, for example, be prepared by admixing the four components and a solvent (for instance, a buffer listed above) to form a mixed solution, which may or may not include a binder such as those listed above, immersing an absorbing support into the mixed solution or applying or coating the solution onto the surface of a support and then drying the support containing the solution or having a layer of the solution thereon. In this method, the support used may be those disclosed before. Moreover, the solution may include an additive other than binders, as those described above.

When preparing these indicating means of the invention, it is preferred to previously produce an indicator having a large dimension and then cut the same into pieces having a desired size (for instance, 5 x 5 mm) and each resulting piece may effectively be used in each inspection.

When carrying out the method according to the present invention, an animal milk as a specimen may be supplied to the inspection procedure without subjecting it to any particular pretreatment. The method of the invention can be performed by, for example, simply dropping one drop of the specimen on the indicator of the invention having the construction described above. In this method, it is sufficient to use only one drop of the specimen. Thereafter, a series of reactions explained above immediately takes place and these reactions are completed within several minutes (usually about 3 minutes). Then, excess amount of the sample is removed by, for example, wiping out with a cloth, absorbent cotton or a filter paper and the amount of $\beta$-hydroxybutyric acid is determined by macroscopically comparing or contrasting the degree of color (color tone) developed on the indicator with the standard color tone table. From the result of the quantitative analysis, one can judge whether the dairy cattle, from which the milk to be examined is milked or collected, suffers from ketosis or not.

The standard color tone table as used herein to determine the amount of $\beta$-hydroxybutyric acid in the specimen can be obtained by preparing a series of $\beta$-hydroxybutyric acid solutions having different concentrations as samples to be inspected, carrying out the color developing test similar to that explained above and arranging the color tone developed on each sample in the order of the $\beta$-hydroxybutyric acid concentration.

According to the method for quantitatively inspecting quality of milk and health of the dairy cattle secreting the milk of the present invention, the inspection of quality of milk and health of the cattle is performed quite simply and there is no need for skillfullness when estimating whether the cattle suffers from ketcsis or not (in other words, whether the cattle is in healthy condition or not). Moreover, it requires only a quite short time to carry out the inspection-diagnosis operations. Therefore, the above mentioned requirements offered by dairy farmers can effectively be satisfied according to the method of this invention.

The method according to the present invention may be applied to dairy cattle, goat, sheep or the like, the milk of which is useful in the human diet. However, the method may also be applied to inspect health of mammals other than those mentioned above so as to protect the mother body thereof or young animals from particularly ketosis.

The reagents as used herein will be explained below more in detail.

$\beta$-hydroxybutyric acid dehydrogenase as used in the method of this invention may be any enzymes which permit the oxidation of $\beta$-hydroxybutyric acid and the conversion of the same to acetoacetic acid and includes, as typical examples, dehydrogenase originated from bacteria such as Rhodopseudomonas spheroides or Pseudomonas lemoignei and those extracted from animal tissues such as heart of cows or liver of pigs.

NAD is a coenzyme serving as a receptor of hydrogen atom and those extracted from animal tissues such as livers or kidneys or microorganisms such as yeast and purified can effectively be used in the method according to the present invention.

The electron carrier is used herein because of its function as an activator of NADH formed during the dehydrogenation of $\beta$-hydroxybutyric acid, the function of NADH as the hydrogen-donner being activated due to the presence of such electron carrier. As the electron carrier, any materials having such function as activator may be used herein and there may be mentioned such as diaphorase which is a kind of flavin enzyme, 9-dimethylaminobenzo-$\alpha$-phenazoxonium chloride (so-called Meldola's Blue), N-methylphenazonium methyl sulfate (the abbreviation thereof being PMS), 1-methoxy-5-methylphenazium methyl sulfate (the abbreviation thereof being 1-methoxy PMS). Among others, it is effective to use 1-methoxy PMS because of its high stability and easy availability with reasonable cost.

The tetrazolium salt as used in the method of this invention includes monotetrazolium salts such as 3-(p-iodophenyl)-2-(p-nitrophenyl)-5-phenyltetrazolium chloride (the abbreviation thereof being INT) and 3-(4,5-dimethyl-2-thiazolyl)-2,5-diphenyl-2H-tetrazolium bromide (the abbreviation thereof being MTT); ditetrazolium salts such as 3,3'-(3,3'-dimethoxy-4,4'-biphenylylene)-bis(2,5-diphenyl-2H-tetrazolium chloride) (so-called TB) and 3,3'-(3,3'-dimethoxy-4,4'-biphenylylene)-bis[2-(p-nitrophenyl l)-5-phenyl-2H-tetrazolium chloride] (so-called nitro-TB). Among others, nitro-TB is the most preferred example of the tetrazolium salt as used herein, because of its high solubility in water and its relatively high stability.

A judgement on the degree of ketosis is given based on the followings.

| Judgement | Concentration of β-hydroxybutyric acid |
|---|---|
| Normal or Health | 0 - 99 (μmol/ℓ) |
| False positive | 100 - 199 |
| Positive | 200 - 499 |
| Bad health | 500 or more |

In the case of the liquid indicator, the amount of β-hydroxybutyric acid dehydrogenase. NAD or the tetrazolium salt usually falls within a range of from 10 to 100 mg (preferably 10 mg), from 20 to 50 mg (preferably 33 mg) or from 10 to 30 mg ( preferably 20 mg), respectively based on 100 ml of buffer solution. While in the case of the solid indicator, the amount thereof is in a range of from 10 to 100 units (preferably 50 units), from 5 to 30 mg (preferably 10 mg) or from 10 to 30 mg (preferably 20 mg) based on the unit surface area of the indicator, respectively.

Moreover, the electron carrier as the activator for NADH may be used in an amount of from 1 to 5 mg (preferably 3 mg) (or 0.5 to 3 mg, preferably 1 mg in the case of the solid indicator).

Each component of the indicator may be used separately or as a combination of at least two compounds. However, if each component is used in a combination, it should be needed to previously prepare the standard color tone table depending on each case according to the same procedures as those explained above or according to the procedures explained below (see Reference Example).

Thus, according to the method of this invention, various kinds of advantages explained below in more detail can easily be attained. First of all, the quantitative inspection of quality of an animal milk and health of the animal secreting the milk can be carried out within an extremely short time, for instance, within approximately 5 minutes and by extremely simple procedures such that only one drop (0.1 to 0.3 ml) of milk is required to effect these inspections. This is resulted from the fact that β-hydroxybutyric acid having a relatively high stability compared with other ketones is selected to determine the physical condition of the animals to be examined and the quality of milk thereof and that a solid and integrated composition (the solid indicator) for detecting the presence of β-hydroxybutyric acid in the samples can be used in the method according to the present invention. Therefore, the method of this invention can be carried out at any time and anywhere the operator wants without using any special test machines.

Thus, the practical milking operations may be effected without any troublesome problems such as already explained above, after conducting a preliminary inspection of milk collected beforehand in a small amount and confirming the animal secreting milk being in the healthy state. This means that dairy farmers can always forward milk of a high quality to the market.

Moreover, in detecting the presence of ketosis state in a subject to be examined according to any one of the conventional methods, the conventional method needs complicated and quite expensive procedures such that a skilled person such as a veterinary must collect or extract samples, for instance, blood or urine of the subject. Under such circumstances, the health inspection of the subject such as dairy cattle had, in general, to rely on the regular medical checkup carried out only once a year. However, the method according to the present invention may be carried out by anybody who must not be a specialist, since the method of this invention is quite simple to carry out and is completed within an extremely short time as explained before. As a result, the animals secreting milk in particular dairy cattle may always be controlled in a n optimum health state and dairy farmers can always forward only the milk obtained from healthy dairy cattle to the market. Moreover, it is also possible to attain secondary effect such that the detection of ketosis in its early stage becomes possible and that the dairy cattle suffering from ketosis can be medical treated in the early stage thereof.

The method according to the present invention will hereunder be explained in more detail with reference to the following nonlimitative and illustrative Examples and Reference Examples.

Reference Example 1 (Relationship Between Reaction Time and Variation in Absorbance and Calibration Curve)

A reaction solution was prepared by dissolving 10 mg of β-hydroxybutyric acid dehydrogenase (derived from Pseudomonas lemoignei: specific activity = 10 U/mg), 33 mg of NAD, 3 mg of 1-methoxy PMS and 17 mg of nitro-TB in 100 ml of 0.1M tris-hydroxyaminomethane-hydrochloric acid buffer (pH: 8.5).

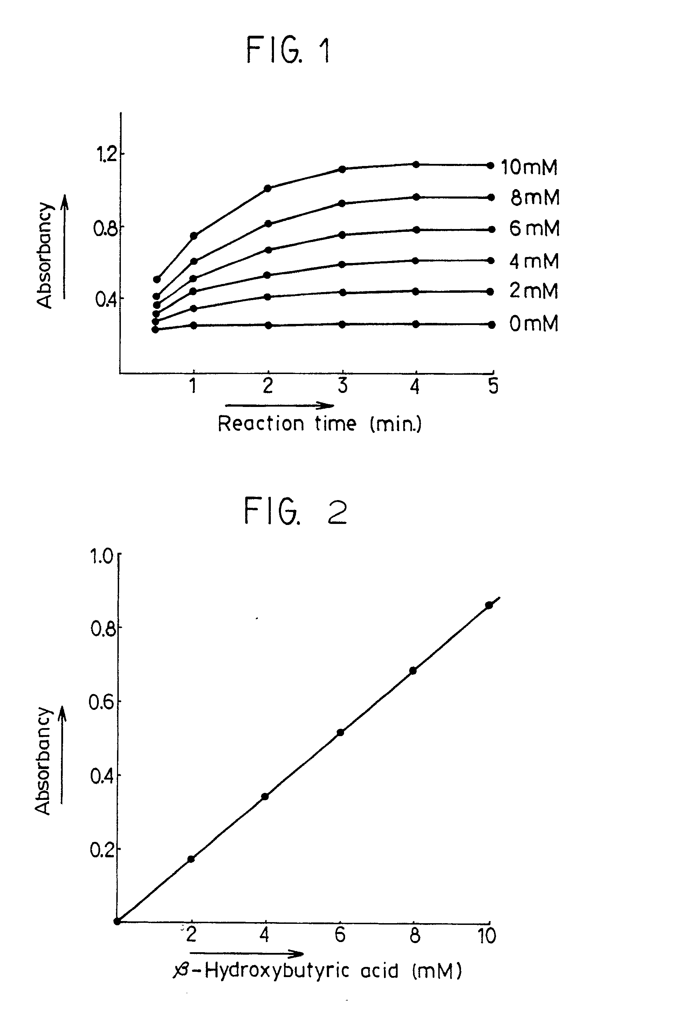

3 ml each of the resulting solution was distributed to a series of test tubes and 2μℓ each of standard solution having a desired concentration of β-hydroxybutyric acid (0, 2, 4, 6, 8 and 10 mM/ℓ) respectively was then added to every test tubes. The test tubes were maintained at 37°C and the absorbancy at 520 mm (monochromatic light) thereof was monitored at each reaction time of 0.5, 1, 2, 3, 4 and 5 minutes utilizing a colorimeter. The results obtained are plotted in Fig. 1. As seen from the results shown in Fig. 1, it was found that the reaction completed approximately after 4 minutes irrespective of the concentration of $\beta$-hydroxybutyric acid present.

Then, the absorbancy corresponding to each standard solution and observed at the reaction time of 4 minutes was plotted against the concentration of the standard $\beta$-hydroxybutyric acid solution (results are shown in Fig. 2), while the absorbancy plotted in Fig. 2 is a reduced value obtained by subtracting the absorbancy of the control (0 mM/$\ell$ of $\beta$-hydroxybutyric acid) from the absorbancy observed on each tube containing $\beta$-hydroxybutyric acid in the predetermined amount. The resulting relationship between the absorbancy and the concentration is almost linear and thus the results can surely be used as the calibration curve in the method according to the invention.

Reference Example 2 (Preparation of Standard Color Tone Table)

A solution was prepared by dissolving 10 mg of NAD, 1 mg of 1-methoxy PMS, 20 mg of nitro-TB, bovine serum albumin and 50 U of $\beta$-hydroxybutyric acid dehydrogenase identical to that used in the Reference Example 1 in 10 ml of 0.1M boric acid-borax buffer (pH: 8.0). A filter paper was then immersed in the solution and dried in the air at room temperature.

The dried filter paper was cut into pieces of 5 mm square and bonded to the head portion of a polyvinylchloride film strip of 5 x 70 mm by the use of a double-coated adhesive tape to obtain a solid indicating means (indicator) according to the present invention.

On the other hand, standard solutions having various concentration of $\beta$-hydroxybutyric acid were prepared (concentration: 0, 30, 50, 80, 100, 130, 150, 180, 200, 230 and 250 $\mu$mol/$\ell$). One drop each of the standard solution was given on the filter paper portion of the solid indicator and maintained for 3 minutes to cause reactions. In these tests, there was observed a color development if $\beta$-hydroxybutyric acid was present and the color tone varied depending on the concentration of $\beta$-hydroxybutyric acid. The resulting solid indicators having different color tone were arranged in the order of the concentration of $\beta$-hydroxybutyric acid and used as the standard color tone Table in the following examples.

Example 1

As test animal, 10 heads (Nos. 1 to 10) of dairy cows which were judged to be normal according to the clinical inspection, 3 heads (Nos. 11 to 13) of dairy cows which were judged to be a mild case of ketosis and 2 heads (Nos. 14 to 15) of dairy cows which were judged to be an advanced case of ketosis were selected. One drop of the milk collected from each test animal as the specimen was dropped on the filter paper portion of the solid indicator prepared in the Reference Example 2 and maintained for 3 minutes. Then, the excess amount of milk was wiped out with absorbent cotton and the resulting color tone of the specimens was macroscopically compared or contrasted with those of the standard color tone Table obtained in the reference exmaple 2. The results are listed in the following Table:

| Test Animal No. | Clinical Diagnosis | | Clinical Esti-mation | Conc. of β-OHBA in Milk (μmol/ℓ) | Quality Inspection of Milk (Alcohol Test) |
|---|---|---|---|---|---|
| | Anorexia | Reduction in Milk Secretion | | | |
| 1 | ± | - | - | 140 | - |
| 2 | ± | - | - | 120 | - |
| 3 | - | - | - | 40 | - |
| 4 | - | - | - | 110 | - |
| 5 | - | - | - | 80 | - |
| 6 | - | - | - | 40 | - |
| 7 | - | - | - | 80 | - |
| 8 | - | ± | - | 140 | - |
| 9 | - | - | - | 100 | - |
| 10 | ± | - | - | 150 | - |
| 11 | + | ± | + | 180 | ± |
| 12 | + | + | + | 200 | + |
| 13 | + | + | + | 180 | + |
| 14 | +++ | + | ++ | 320 | ++ |
| 15 | +++ | +++ | +++ | 530 | ++ |

Clinical Diagnosis: Anorexia and Reduction in Milk Secretion

-: Not observed

±: Observed but in an extremely small degree

+: Observed in some degree

++: Observed in high degree

+++: Observed in extremely high degree

Clinical Estimation:

-: Judged to be normal

+: Judged to be ketosis (a mild case)

++: Judged to be ketosis (an advanced case)

+++: Judged to be ketosis (an advanced case)

Example 2

Test animals (dairy cows) were randomly selected and the milk, as the sample, obtained from each test animal was immediately subjected to the inspection according to the same procedures as disclosed in the Example 1.

Then, the alcohol test (test for thermal stability) and acidity test were carried out on 10 samples of milk containing β-hydroxybutyric acid in an amount less than 180 μmol/ℓ and 3 samples of milk containing equal to or more than 300 μmol/ℓ thereof selected according to the test mentioned above in order to estimate the quality grade of these samples. As a result, the former were judged to be the first grade, while the latter were classified to be the secondary grade.

This fact means that the method according to the present invention is one of the most effective methods for estimating the quality of milk.

**Claims**

1. A method for simultaneously inspecting the quality of the milk secreted by an animal and diagnosing whether the animal is in a state of ketosis, which comprises collecting milk from the animal to be inspected as a specimen, dehydrogenating β-hydroxybutyric acid included in the specimen with a β-hydroxybutyric acid dehydrogenase under the presence of nicotinamide adenine dinucleotide to convert it to acetoacetic acid, adding and reducing a tetrazolium salt with nicotinamide adenine dinucleotide in the reduced form formed during the above dehydrogenation through the action of an electron carrier to form a colored formazan and then determining the amount of β-hydroxybutyric acid included in the specimen based on the degree of color development or color tone of the colored formazan.

2. The method as set forth in Claim 1, wherein the determination of the amount of β-hydroxybutyric acid in the specimen is performed by macroscopically comparing or contrasting the color tone of the resulting formazan with those of a preliminary prepared standard color tone table.

3. The method as set forth in Claim 1 or 2, wherein the specimen is directly treated with a mixture of the β-hydroxybutyric acid dehydrogenase, nicotinamide adenine dinucleotide, the electron carrier and the tetrazolium salt.

4. The method as set forth in Claim 1, wherein the β-hydroxybutyric acid dehydrogenase is a member selected from the group consisting of those originated from Rhodopseudomonas spheroides and Pseudomonas lemoignei and those extracted from animal tissue.

5. The method as set forth in Claim 1, wherein the electron carrier is a member selected from the group consisting of diaphorase, 9-dimethylaminobenzo-α-phenazoxonium chloride, N-methylphenazonium methyl sulfate, 1-methoxy-5-methylphenazium methyl sulfate.

6. The method as set forth in Claim 1, wherein the tetrazolium salt is a member selected from the group consisting of 3-(p-iodophenyl)-2-(p-nitrophenyl)-5-phenyltetrazolium chloride, 3-(4,5-dimethyl-2-thiazolyl)-2,5-diphenyl-2H-tetrazolium bromide, 3,3'-(3,3'-dimethoxy-4,4'-biphenylylene)-bis(2,5-diphenyl-2H-tetrazolium chloride) and 3,3'-(3,3'-dimethyl-4,4'-biphenylylene)-bis[2-(p-nitrophenyl)-5-phenyl-2H-tetrazolium chloride].

**Revendications**

1. Procédé pour contrôler simultanément la qualité du lait secrété par un animal et pour diagnostiquer si l'animal est dans un état de cétose, comprenant la collecte du lait provenant de l'animal à contrôler sous forme d'un échantillon, la deshydrogénation de l'acide β-hydroxybutyrique inclus dans l'échantillon par une déshydrogénase de l'acide β-hydroxybutyrique, en présence de nicotinamide adénine dinucléotide pour le convertir en acide acétylacétique, l'addition et la réduction d'un sel de tétrazolium avec la nicotinamide adénine dinucléotide sous forme réduite, formée durant ladite déshydrogénation par l'action d'un donneur d'électrons pour former un formazan coloré et ensuite la détermination de la quantité d'acide β-hydroxybutyrique inclus dans l'échantillon, en se basant sur le degré du développement de la couleur ou sur la teinte du formazan coloré.

2. Procédé selon la revendication 1, caractérisé en ce que la détermination de la quantité d'acide β-hydroxybutyrique dans l'échantillon est réalisée de façon macroscopique en comparant ou en observant le contraste entre la teinte du formazan et celles d'une table de teintes témoins préparées au préalable.

3. Procédé selon la revendication 1 ou 2, caractérisé en ce que l'échantillon est traité directement avec un mélange de déshydrogénase de l'acide β-hydroxybutyrique, de nicotinamide adénine dinucléotide, de porteur d'électrons et de sel de tétrazol.

4. Procédé selon la revendication 1, caractérisé en ce que la déshydrogénase de l'acide β-hydroxybutyrique est un élément choisi parmi le groupe constitué de celles provenant de Rhodopseudomas

EP 0 226 427 B1

spheroides et Pseudomonas lemoignei et de celles extraites du tissu animal.

5. Procédé selon la revendication 1, caractérisé en ce que le porteur d'électrons est un élément choisi parmi le groupe comprenant la diaphorase, le chlorure de 9-diméthylaminobenzo-α-phénazoxonium, le méthyl sulfate de N-méthylphénazonium, ou le méthyl sulfate de 1-méthoxy-5-méthylphénazonium.

6. Procédé selon la revendication 1, caractérisé en ce que le sel de tétrazol est un élément choisi parmi le groupe comprenant le chlorure de 3-(p-iodophényl) - 2 - (p-nitrophényl) - 5 - phényltétrazol, le bromure de 3 - (4, 5 - diméthyl - 2 - thiazolyl) - 2,5 - diphényl - 2H - tétrazol, le 3,3' - (3,3' - diméthoxy - 4,4' - biphénylylène) - bis (chlorure de 2,5 - diphényl - 2H - tétrazol) et le 3,3' - (3,3'- diméthyl - 4,4' - biphénylylène) - bis - (chlorure de 2 - (p - nitrophényl) - 5 - phényl - 2H - tétrazol.

**Patentansprüche**

1. Verfahren zum Testen der Qualität von tierischer Milch und zum gleichzeitigen Diagnostizieren eines eventuellen Ketosezustandes des Tieres, umfassend die Entnahme einer Milchprobe von dem zu untersuchenden Tier, Dehydrogenisieren der in der Probe enthaltenen ß-Hydroxybuttersäure mit ß-Hydroxybuttersäure-Dehydrogenase in Anwesenheit von Nikotinamidadenindinukleotid zu deren Umwandlung zu Acetessigsäure, Hinzugeben und Reduzieren eines Tetrazolsalzes mit Nikotinamidadenindinukleotid in der während der genannten Dehydrogenisierung durch die Wirkung eines Elektronenträgers gebildeten reduzierten Form zur Bildung von gefärbtem Formazan und anschließendes Bestimmten der Menge an in der Probe enthaltener ß-Hydroxybuttersäure nach dem Ausmaß der Farbentwicklung bzw. des Farbtones des gefärbten Formazans.

2. Verfahren nach Anspruch 1, wobei die Bestimmung der in der Probe enthaltenen Menge an ß-Hydroxybuttersäure durch makroskopisches Vergleichen oder Kontrastieren des Farbtones des erhaltenen Formazans mit den Farbtönen auf einer zuvor vorbereiteten Standardfarbtontabelle erfolgt.

3. Verfahren nach Anspruch 1 oder 2, wobei die Probe unmittelbar mit einem Gemisch aus ß-Hydroxybuttersäure-Dehydrogenase, Nikotinamidadenindinukleotid, Elektronenträger und Tetrasolsalz behandelt wird.

4. Verfahren nach Anspruch 1, wobei die ß-Hydroxybuttersäure-Dehydrogenase aus einer Gruppe bestehend aus Rhodopseudomonas spheroides und Pseudomonas lemoignei und aus Tiergewebeextrakten ausgewählt wurde.

5. Verfahren nach Anspruch 1, wobei der Elektronenträger aus einer Gruppe bestehend aus Diaphorase, 9-Dimethylaminobenzo-α -Phenazoxonchlorid, N-Methylphenazon-Methylsulfat, 1-Methoxy-5-Methylphenazium-Methylsulfat ausgewählt wurde.

6. Verfahren nach Anspruch 1, wobei das Tetrazolsalz aus einer Gruppe bestehend aus 3-(p-Jodphenyl)-2-(p-Nitrophenyl)-5-Phenyltetrazolchlorid, 3-(4,5-Dimethyl-2-Thiazolyl)-2,5-Diphenyl-2H-Tetrazolbromid, 3,3'-(3,3'-Dimethoxy-4,4'-Biphenylylen)-bis(2,5-Diphenyl-2H-Tetra -zolchlorid) und 3,3'-(3,3'-Dimethyl-4,4'-Biphenylylen)-bis[2-(p-Nitrophenyl)-5-Phenyl-2H-Tetrazolchlorid] ausgewählt wurde.

# FIG. 1

# FIG. 2